# EUROPEAN PATENT APPLICATION

(11) **EP 4 534 096 A1**
(43) Date of publication of application: **09.04.2025**
(21) Application number: 24201499.1
(22) Date of filing: 19.09.2024
(51) Int. Cl.: A61K 38/16, A61P 3/06, A61P 3/10, A61K 36/42

(54) **POLYPEPTIDE AND USE THEREOF IN REGULATING BLOOD GLUCOSE LEVEL AND/OR REDUCING FAT**

(30) Priority: 03.10.2023 TW 112137950
(71) Applicant: Greenyn Biotechnology Co., Ltd, Taichung City (TW)
(72) Inventor: Hsu, Pang-Kuei, Taichung City (TW); Lin, Yu Cheng, Taichung City (TW); Wu, Chia-Feng, Taichung City (TW)
(74) Representative: Lang, Christian

(57) **Abstract**

A polypeptide and a use thereof in regulating blood glucose level and/or reducing fat are disclosed. The amino acid sequence of the polypeptide includes the sequence of SEQ ID No.: 1, the sequence of SEQ ID No.: 2, the sequence of SEQ ID No.: 3, the sequence of SEQ ID No.: 4, or a sequence obtained by modifying at least one amino acid in any of the aforesaid sequences. The polypeptide disclosed herein has the physiological activity to enhance fat metabolism and regulate blood glucose level. Therefore, administering an effective amount of the polypeptide, or a composition containing the polypeptide, to an individual can effectively produce the effect of treating or preventing a disease related to blood glucose imbalance or to imbalance in fat metabolism.

## Description

### BACKGROUND OF THE INVENTION

### 1. Technical Field

The present invention relates to a polypeptide and a use thereof. More particularly, the invention relates to a polypeptide and a use thereof in regulating blood glucose level and/or reducing fat.

### 2. Description of Related Art

According to a survey, the prevalence rate of diabetes among people not younger than 18 years old in Taiwan has kept increasing and has been higher than the global average, and there has been a trend for the ages of patients with diabetes to decrease. Apart from being associated with genetics, the onset of diabetes is mostly caused by an eating habit that involves highly refined, high-sugar, and high-fat food. As it is difficult to change one's eating habit, the market has been supplied with numerous nutritional supplements that are related to blood glucose regulation and weight reduction. The main active ingredients of many of those nutritional supplements are peptides.

Peptides are structures composed of amino acids. Peptides are substances larger than amino acids and smaller than proteins, have much lower molecular weights than proteins, and therefore can be directly absorbed, or have a higher absorption rate than proteins, after entering the human body. Moreover, many peptides were found in recent studies to have specific physiological functions or activity, such as to prevent osteoporosis, inhibit cancer, and fight against aging. This is why peptides are now widely used in nutritional supplements and skin care products. Currently, peptides are in most cases obtained by performing enzymatic hydrolysis on proteins. While enzymatic hydrolysis can produce peptides rapidly, it remains a major challenge to carry out quality control and increase product purity.

### BRIEF SUMMARY OF THE INVENTION

The primary objective of the present invention is to provide a polypeptide and a use thereof in regulating blood glucose level and/or reducing fat. More specifically, the polypeptide disclosed herein has the ability to activate an insulin receptor, to regulate blood glucose level by encouraging cells to perform active transport of glucose, to enhance the oxidation and metabolism of adipocytes, and to thereby effectively produce the effect of regulating blood glucose level and/or preventing fat build-up.

The secondary objective of the present invention is to provide a polypeptide and a use thereof in regulating blood glucose level and/or reducing fat, wherein the polypeptide can regulate blood glucose level and enhance the metabolism of fat at the same time and is therefore capable of regulating multiple metabolic indicators and thereby effectively producing the effect of preventing or treating metabolic syndrome.

To achieve the foregoing objectives, the polypeptide disclosed herein has an amino acid sequence that includes a sequence of SEQ ID No.: 1, a sequence of SEQ ID No.: 2, a sequence of SEQ ID No.: 3, a sequence of SEQ ID No.: 4, or a sequence obtained by modifying one or more of the amino acids in any of the aforesaid sequences.

In one embodiment of the present invention, the amino acid sequence of the polypeptide is SEQ ID No.: 1.

In another embodiment of the present invention, the amino acid sequence of the polypeptide is SEQ ID No.: 2.

In still another embodiment of the present invention, the amino acid sequence of the polypeptide is SEQ ID No.: 3.

In yet another embodiment of the present invention, the amino acid sequence of the polypeptide is SEQ ID No.: 4.

The polypeptide disclosed herein can be obtained by artificial synthesis, enzymatic hydrolysis, solvent extraction, or other protein preparation techniques that are well known in the art. For example, the polypeptide as the amino acid sequence of SEQ ID No.: 4 may be obtained by artificial synthesis or by performing an enzymatic hydrolysis procedure on the polypeptide as the amino acid sequence of SEQ ID No.: 3.

Furthermore, as the polypeptide disclosed herein has the function of regulating blood glucose level and/or enhancing the metabolism of fat, one embodiment of the invention uses the disclosed polypeptide to prepare a composition for regulating blood glucose level. That is to say, when administered in an effective amount to an individual who has a symptom of a blood glucose regulation-related disease or who already has a disease related to imbalance in blood glucose metabolism, the polypeptide disclosed herein can activate an insulin receptor, encourage cells to perform active transport of glucose, and thus effectively produce the effect of regulating the individual's blood glucose level and delaying the onset of a blood glucose-related disease.

The composition disclosed herein for regulating blood glucose level includes the polypeptide as SEQ ID No.: 1, the polypeptide as SEQ ID No.: 2, and the polypeptide as SEQ ID No.: 3.

Alternatively, the composition disclosed herein for regulating blood glucose level includes the polypeptide as SEQ ID No.: 1, the polypeptide as SEQ ID No.: 2, the polypeptide as SEQ ID No.: 3, and the polypeptide as SEQ ID No.: 4.

In another embodiment of the present invention, the polypeptide disclosed herein is used to prepare a composition for reducing fat, or more particularly for enhancing the activity of an individual's cells to metabolize fat so that even if the individual has a high-fat or high-calorie diet, the composition can still inhibit the formation of body fat and prevent fat from accumulating in the individual's body, thereby effectively producing the effect of reducing fat and body weight.

The composition disclosed herein for reducing fat includes the polypeptide as SEQ ID No.: 1, the polypeptide as SEQ ID No.: 2, and the polypeptide as SEQ ID No.: 3.

Alternatively, the composition disclosed herein for reducing fat includes the polypeptide whose amino acid sequence is SEQ ID No.: 1, the polypeptide whose amino acid sequence is SEQ ID No.: 2, the polypeptide whose amino acid sequence is SEQ ID No.: 3, and the polypeptide whose amino acid sequence is SEQ ID No.: 4.

Another embodiment of the present invention discloses a composition that includes an effective amount of the foregoing polypeptide, wherein the composition may be, for example, a pharmaceutical, a food, or a nutritional supplement. When administered to an individual, the composition can produce the effect of treating or preventing a disease caused by blood glucose imbalance or by imbalance in fat metabolism. Diseases caused by blood glucose imbalance or by imbalance in fat metabolism include hyperglycemia, obesity, diabetes, and metabolic syndrome, among others.

### BRIEF DESCRIPTION OF THE SEVERAL VIEWS OF THE DRAWINGS

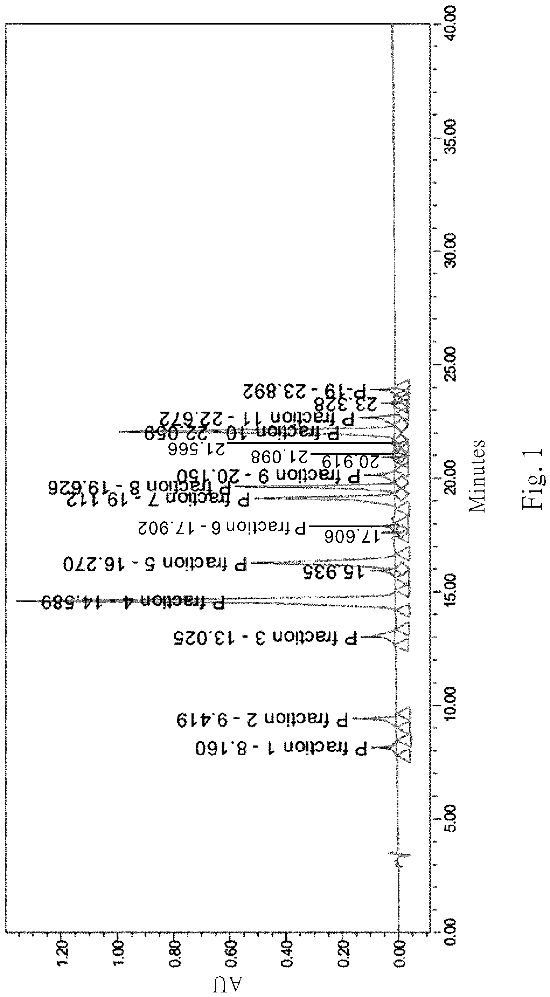

FIG. 1 is a liquid chromatogram of the polypeptide extract disclosed in example 1 of the present invention.

### DETAILED DESCRIPTION OF THE INVENTION

The present invention discloses a polypeptide that can be used to regulate blood glucose level and/or reduce fat, and the amino acid sequence of the polypeptide includes the sequence of any of SEQ ID No.: 1 to SEQ ID No.: 4 (as shown in Table 1 below) or a sequence obtained by modifying one or more amino acids in any of the sequences of SEQ ID No.: 1 to SEQ ID No.: 4. The polypeptide disclosed herein has such functions as activating an insulin receptor, encouraging cells to carry out active transport of glucose, and enhancing the oxidation and metabolism of adipocytes. Therefore, administering an effective amount of the disclosed polypeptide, or a composition containing the polypeptide, to an individual can effectively produce the effect of reducing fat and/or regulating blood glucose level and thereby prevent or treat metabolic syndrome, hyperglycemia, diabetes, and other diseases related to abnormality in blood glucose or fat metabolism.

**Table 1: Amino acid sequences of the polypeptides disclosed in the present invention**

| Name of peptide | Amino acid sequence number | Amino acid sequence |
|---|---|---|
| Polypeptide 1 | SEQ ID No.: 1 | GGGDATRYSTGGGYS |
| Polypeptide 2 | SEQ ID No.: 2 | INPNEQREV |
| Polypeptide 3 | SEQ ID No.: 3 | MQIFVKTLTG |
| Polypeptide 4 | SEQ ID No.: 4 | MQIFVK |

In one embodiment of the present invention, the amino acid sequence of the polypeptide disclosed herein for regulating blood glucose level and/or reducing fat is as SEQ ID No.: 1, SEQ ID No.: 2, SEQ ID No.: 3, or SEQ ID No.: 4.

As used herein, the term "modify" refers to performing substitution, deletion, or insertion at least one amino acid site in an amino acid sequence.

As used herein, the term "polypeptide" refers to a small-molecule protein composed of a plurality of amino acids.

The polypeptide disclosed herein can be prepared by artificial synthesis, enzymatic hydrolysis, or other techniques that are well known in the art to be able to produce peptides. The so-called artificial synthesis includes, for example, chemical synthesis and biological synthesis. Chemical synthesis involves connecting a plurality of amino acids with peptide bonds in order to obtain the target peptide and can be further divided by the synthesis technique into liquid-phase synthesis, solid-phase synthesis, and so on. Biological synthesis, on the other hand, uses a recombinant microorganism or recombinant cell to produce the target peptide. More specifically, a nucleic acid molecule in the recombinant microorganism or cell that can express the target peptide is transfected into a microorganism or cell by a biological technique.

The polypeptide disclosed herein exists in animals or plants and therefore can be separated from a raw material such as the fruit, fruit skin, fruit flesh, seeds, and/or leaves of pumpkin, watermelon, cucumber, sponge gourd, or other plants of the family Cucurbitaceae by extraction or other separation techniques well known in the art.

The polypeptide disclosed herein can also be obtained by purifying a plant protein or animal protein such as pumpkin protein, sponge gourd protein, cucumber protein, or the proteins of other plants of the family Cucurbitaceae. Common conventional protein purification techniques include salt fractionation, gel filtration, chromatography, and so on. A plant protein or animal protein may be subjected to a procedure such as enzymatic hydrolysis or extraction, before protein purification is performed, i.e., before a protein purification technique is used, to obtain the target peptide.

As used herein, the term "composition" refers to a substance containing an effective amount of the polypeptide disclosed herein, such as a nutritional supplement, a pharmaceutical, or a food, wherein the so-called effective amount is in the range from 0.01% to 100% of the total weight of the composition. The dosage form of the composition may be, for example but not limited to, powder, tablets, drops, or liquid. Moreover, the composition may further include at least one ingredient. For example, the composition may include the polypeptide disclosed herein and at least one pharmaceutically acceptable vehicle, or the composition may include the polypeptide disclosed herein and at least one ingredient that is acceptable in food.

To better demonstrate the technical features of the present invention and their effects, a few experimental examples and their data are provided below.

The polypeptides used in the following examples were obtained by artificial synthesis, and their sequences have been identified and verified by high-performance liquid chromatography (HPLC) and liquid chromatography-mass spectrometry (LC-MASS). A total of five polypeptides were used in the following examples. The amino acid sequences of the polypeptides are the sequences of SEQ ID No.: 1 to SEQ ID No.: 5, and the molecular weights of the polypeptides are shown in Table 2.

**Table 2: Molecular weights of various polypeptides**

| Name of peptide | Amino acid sequence number | Molecular weight | Amino acid sequence |
|---|---|---|---|
| Polypeptide 1 | SEQ ID No.: 1 | 1405.40 | GGGDATRYSTGGGYS |
| Polypeptide 2 | SEQ ID No.: 2 | 1098.53 | INPNEQREV |
| Polypeptide 3 | SEQ ID No.: 3 | 1404.59 | MQIFVKTLTG |
| Polypeptide 4 | SEQ ID No.: 4 | 765.62 | MQIFVK |
| Polypeptide 5 | SEQ ID No.: 5 | 841.53 | VATVIQVL |

The cells used in the following examples were commercial cell lines and therefore need not be deposited.

### Example 1: Preparation of a polypeptide extract

Pumpkin seeds were obtained and were subjected to such pretreatment procedures as washing and grinding into powder, before an extraction procedure was performed. The extraction procedure may be carried out by any extraction technique that is well known to a person of ordinary skill in the art, such as solvent extraction, supercritical fluid extraction, or supersonic extraction. In this example, the extraction procedure was performed by supercritical fluid extraction, using carbon dioxide as the solvent, with the extraction temperature being 30-80°C, and the pressure being 8.2-50.0 MPa. When the extraction was completed, a crude extract was obtained. The crude extract went through such procedures as disinfection, drying, and pulverization to produce the polypeptide extract of the present invention, whose liquid chromatogram is shown in FIG. 1.

According to FIG. 1, the polypeptide extract of the present invention peaked at the following retention time: 8.1-8.2, 9.4-9.5, 13.0-13.1, 14.5-14.6, 16.2-16.3, 17.9-18.0, 19.6-19.7, 20.1-20.2, 22.0-22.1, and 22.6-22.7 minutes. This indicates that the polypeptide extract of the invention was indeed a mixture of multiple peptides. More specifically, peptide fraction 1 (P fraction 1) corresponded to the retention time of 8.160 minutes, peptide fraction 2 (P fraction 2) corresponded to the retention time of 9.419 minutes, peptide fraction 3 (P fraction 3) corresponded to the retention time of 13.025 minutes, peptide fraction 4(P fraction 4) corresponded to the retention time of 14.589 minutes, peptide fraction 5 (P fraction 5) corresponded to the retention time of 16.270 minutes, peptide fraction 6 (P fraction 6) corresponded to the retention time of 17.902 minutes, peptide fraction 7 (P fraction 7) corresponded to the retention time of 19.112 minutes, peptide fraction 8 (P fraction 8) corresponded to the retention time of 19.626 minutes, peptide fraction 9 (P fraction 9) corresponded to the retention time of 20.150 minutes, peptide fraction 10 (P fraction 10) corresponded to the retention time of 22.059 minutes, peptide fraction 11 (P fraction 11) corresponded to the retention time of 22.672 minutes, and peptide fraction 19 (P fraction 19) corresponded to the retention time of 23.892 minutes.

### Example 2: Animal test (1)

A plurality of 8-week-old Sprague-Dawley (SD) rats were obtained, randomly divided into two groups, and intraperitoneally injected with the drug streptozotocin (STZ) at 55-75 mg/kg in order to induce type-1 diabetes and thereby create a type-1 diabetes model. One week after the induction, and while the rats were fasting, blood samples were taken for a blood glucose level test. The induction was deemed successful when the blood glucose level was higher than 230 mg/dL, and no more injection of the drug STZ was necessary when the induction was successful. The rats in the blank group were fed with high-calorie feed after being injected with the drug STZ. The rats in the polypeptide group, however, were fed with both high-calorie feed and the polypeptide extract in example 1 after being injected with the drug STZ, wherein the dosage of the polypeptide was converted from the human dosage of 300 mg/day, i.e., the dosage for the rats was 31.2 mg/kg (which was obtained by calculating 300/60*6.25).

The rats in both groups were raised under the foregoing conditions for 8 weeks and then had their blood glucose levels and glycated hemoglobin (HbA1c) levels tested. The test results are shown in Table 3.

**Table 3: Blood glucose levels and glycated hemoglobin levels of the rats in both groups after the test**

| Group | Blood glucose level (mg/dL) | Glycated hemoglobin level (%) |
|---|---|---|
| Blank group | 259 ±18.1 | 9.7 ±2.6 |
| Polypeptide group | 170 ±18.4 | 10.2 ±1.1 |

It can be known from the results in Table 3 that the rats in the polypeptide group had significantly lower (about 40% lower) blood glucose levels and lower glycated hemoglobin levels than the rats in the blank group. It can be inferred from the results that the polypeptide extract of the present invention can effectively regulate an individual's blood glucose level, and that even when the individual's pancreatic islets are damaged, the polypeptide extract can still effectively lower the chance of diabetes developing in the individual.

### Example 3: Animal test (2)

A plurality of 8-week-old SD rats were obtained, randomly divided into two groups, and intraperitoneally injected with the drug STZ at a low dosage every three days in order to induce type-2 diabetes and thereby create a type-2 diabetes model. During the induction (i.e., injection), in every week the blood samples were taken for testing the fasting blood sugar. The induction was deemed successful when the blood glucose level was higher than 230 mg/dL, and no more injection of the drug STZ was necessary when the induction was successful.

The rats in both groups were fed with high-calorie feed after the injection began. The rats in one of the groups (hereinafter referred to as the polypeptide group) were also given the polypeptide extract in example 1, and the polypeptide extract was administered at a dosage converted from the human dosage of 300 mg/day, i.e., the dosage for the rats was 31.2 mg/kg (which was obtained by calculating 300/60*6.25). The rats in both groups were raised under the foregoing conditions for 8 weeks and then had their blood glucose levels and glycated hemoglobin (HbA1c) levels tested. The test results are shown in Table 4.

**Table 4: Blood glucose levels and glycated hemoglobin levels of the rats in both groups after the test**

| Group | Blood glucose level (mg/dL) | Glycated hemoglobin level (%) |
|---|---|---|
| Blank group | 222 ±30.9 | 9.28 ±1.8 |
| Polypeptide group | 145 ±14.6 | 7.95 ±0.9 |

It can be known from the results in Table 4 that the rats in the polypeptide group had significantly lower (about 38.8% lower) blood glucose levels and lower (about 14.3% lower) glycated hemoglobin levels than the rats in the blank group. It can be inferred from the results that when an individual has, or is at high risk of having, type-2 diabetes, administering the polypeptide extract of the present invention to the individual can effectively regulate the individual's blood glucose level, thereby producing the effect of preventing, or delaying the onset of, diabetes or a disease related to imbalance in blood glucose metabolism.

### Example 4: Animal test (3)

A plurality of 8-week-old SD rats were obtained and randomly divided into two groups. The rats in the blank group were fed only with high-calorie feed, whereas the rats in the polypeptide group were fed with both high-calorie feed and the polypeptide extract in example 1, wherein the polypeptide extract was administered at a dosage converted from the human dosage of 300 mg/day, i.e., the dosage for the rats was 31.2 mg/kg (which was obtained by calculating 300/60*6.25).

The rats in both groups were raised under the foregoing conditions for 8 weeks and then had their body weights measured. The rats were subsequently sacrificed, and their abdominal fat was weighed. The test results are shown in Table 5.

**Table 5: Body weights and abdominal fat weights of the rats in both groups after the test**

| Group | Body weight (g) | Abdominal fat weight (g) |
|---|---|---|
| Blank group | 648.4 ±79.08 | 38.92 ±9.57 |
| Polypeptide group | 532.6 ± 89.23 | 28.12 ±10.12 |

It can be known from the results in Table 5 that the rats in the polypeptide group had significantly lower (17.9% lower) body weights and significantly lower (27.7% lower) abdominal fat weights than the rats in the blank group.

It can be inferred from the foregoing results that administering the polypeptide extract of the present invention to an individual maintaining a high-fat or high-calorie diet not only can inhibit the accumulation of fat, but also can enhance the metabolism of fat to reduce weight, reduce body fat, or prevent a significant increase in body weight. In other words, the polypeptide extract of the invention can be used to prevent and/or treat diseases related to fat metabolism, such as obesity and metabolic syndrome.

### Example 5: Cell test

It can be known from the previous examples that the polypeptide extract in example 1 had the activity to lower the glycated hemoglobin level, regulate blood glucose level, increase the metabolism of fat, and so on. Therefore, polypeptides were separated from the polypeptide extract, sequenced, and identified by SEQ ID No.: 1 to SEQ ID No.: 5. The same polypeptides were then prepared by artificial synthesis and used in the following cell test.

C2C12 mouse myoblasts were obtained and were cultured in groups for 24 hours. After that, the culture medium of each group was added with human insulin (MedChemExpress LLC), polypeptide 1 of the present invention (SEQ ID No.: 1), polypeptide 2 of the invention (SEQ ID No.: 2), polypeptide 3 of the invention (SEQ ID No.: 3), polypeptide 4 of the invention (SEQ ID No.: 4), or polypeptide 5 (whose amino acid sequence number is SEQ ID No.: 5 and which was used for the control group) such that the final concentration of the added insulin/polypeptide in the culture medium was 20 µL/mL. The cells in each group were then cultured for another 24 hours, before the culture medium of each cell group was removed. The cells were subsequently washed with a phosphate-buffered solution, added with a radioimmunoprecipitation assay (RIPA) buffer containing a phosphatase inhibitor, and kept in a refrigerator, allowing reactions to take place for 30 minutes. Following that, the cells were thawed, subjected to vibration extraction with an ultrasonic vibrator for 5 minutes, and centrifuged. The supernatant of each cell group was collected and was analyzed by the western blotting method in order to determine changes in the expression of *p*-IR/IR, *p*-Akt, GLUT4, mTOR, PPARα, and *p*-AMPK in each cell group. The analysis results are shown in Table 6.

**Table 6: Changes in the expression of proteins in each cell group that are related to blood glucose level regulation, insulin receptor activation, and the oxidation and metabolism of fat**

| Cell treatment | *p*-IR/IR | *p*-Akt | GLUT4 | PGC-1α | mTOR | PPARα | *p*-AMPK |
|---|---|---|---|---|---|---|---|
| Adding with polypeptide 1 | 1.81 | +196% | +376% | +161% | +136% | +173% | +232% |
| Adding with polypeptide 2 | 1 | +29% | +23% | +0% | +22% | +62% | +70% |
| Adding with polypeptide 3 | 3.08 | +153% | +582% | +258% | +117% | +196% | +210% |
| Adding with polypeptide 4 | 1.97 | -3% | +156% | +155% | +74% | +151% | +91% |
| Adding with polypeptide 5 | 1 | -42% | +34% | +3% | +0 | +23% | +7% |
| Adding with insulin | 1.23 | +56% | +41% | +50% | -2% | +40% | +1% |

It can be known from the results in Table 6 that polypeptide 1 of the present invention (whose amino acid sequence is SEQ ID No.: 1), polypeptide 2 of the invention (whose amino acid sequence is SEQ ID No.: 2), and polypeptide 3 of the invention (whose amino acid sequence is SEQ ID No.: 3) were able to encourage insulin receptor phosphorylation, increase the expression of phosphorylated Akt and GLUT4, and enhance the expression of PGC-1α and PPARα (which are proteins critical to the fat oxidation pathway) without induction by insulin; that despite failure to increase the expression of phosphorylated Akt significantly, polypeptide 4 of the invention (whose amino acid sequence is SEQ ID No.: 4) was still able to encourage insulin receptor phosphorylation, increase the expression of GLUT4, and enhance the expression of PGC-1α and PPARα (which are proteins critical to the fat oxidation pathway) without induction by insulin; and that polypeptide 5 of the invention (whose amino acid sequence is SEQ ID No.: 5) failed to increase the expression of phosphorylated Akt and had a weaker enhancing effect on the expression of GLUT4, PGC-1α, and PPARα than the polypeptides each of whose amino acid sequences is one of SEQ ID No.: 1 to SEQ ID No.: 4. The results also show that each polypeptide disclosed herein having the amino acid sequence of one of SEQ ID No.: 1 to SEQ ID No.: 4 performed better than the insulin in terms of their respective effects on the expression of *p*-IR/IR, *p*-Akt, GLUT4, mTOR, PPARα, and*p*-AMPK.

It can be inferred from the results in Table 6 that the polypeptides of the present invention, i.e., the polypeptides each having the amino acid sequence of one of SEQ ID NO.: 1 to SEQ ID No.: 4, can activate an insulin receptor, regulate blood glucose level by encouraging cells to perform active transport of glucose, enhance the oxidation and metabolism of adipocytes, and thus effectively produce the effect of regulating an individual's blood glucose level and/or reducing the individual's body weight. In other words, administering an effective amount of any polypeptide having the amino acid sequence of one of SEQ ID NO.: 1 to SEQ ID No.: 4, or a composition containing any of the polypeptides, to an individual can treat and/or prevent diseases related to blood glucose imbalance or to abnormality in fat metabolism, such as diabetes, hyperglycemia, obesity, diabetes, and metabolic syndrome.

## Claims

1. A polypeptide, having an amino acid sequence comprising a sequence of SEQ ID No.: 1, a sequence of SEQ ID No.: 2, a sequence of SEQ ID No.: 3, a sequence of SEQ ID No.: 4, or a sequence obtained by modifying one or more amino acids in any of the aforesaid sequences.

2. The polypeptide of claim 1, wherein the amino acid sequence is as SEQ ID No.: 1, SEQ ID No.: 2, SEQ ID No.: 3, or SEQ ID No.: 4.

3. A composition, comprising an effective amount of a polypeptide, wherein the polypeptide has an amino acid sequence selected from the group consisting of SEQ ID No.: 1, SEQ ID No.: 2, SEQ ID No.: 3, and SEQ ID No.: 4.

4. The composition of claim 3, **characterized in that** the composition comprises the polypeptide as SEQ ID No.: 1, the polypeptide as SEQ ID No.: 2, the polypeptideas SEQ ID No.: 3, and the polypeptide as SEQ ID No.: 4.

5. A use of the polypeptide of claim 2 in preparing a composition for regulating blood glucose level.

6. The use of claim 5, wherein the composition comprises the polypeptide as SEQ ID No.: 1, the polypeptide as SEQ ID No.: 2, the polypeptide as SEQ ID No.: 3, and the polypeptide as SEQ ID No.: 4.

7. A use of the polypeptide of claim 2 in preparing a composition for reducing fat.

8. The use of claim 7, wherein the composition comprises the polypeptide as SEQ ID No.: 1, the polypeptide as SEQ ID No.: 2, the polypeptide as SEQ ID No.: 3, and the polypeptide as SEQ ID No.: 4.
